# EUROPEAN PATENT APPLICATION

(11) **EP 4 834 378 A1**
(43) Date of publication of application: **04.11.2026**
(21) Application number: 25382443.7
(22) Date of filing: 30.04.2025
(51) Int. Cl.: A61L 9/12, A61L 9/14

(54) **DEVICE FOR DIFFUSING VOLATILE SUBSTANCES**

(71) Applicant: Zobele Holding S.P.A., 38123 Trento (IT)
(72) Inventor: DEFLORIAN, Stefano, 38123 TRENTO (IT); SORDO, Walter, 38123 TRENTO (IT)
(74) Representative: Herrero & Asociados, S.L.

(57) **Abstract**

The device for diffusing volatile substances, comprising: a housing (1) provided with an air inlet (3) and a first and second air outlets (4, 5); a fan (2) placed inside the housing (1) for directing air from the air inlet (3) to the air outlets (4, 5); and a container (10) housing liquid with volatile substances, the container (10) being connected to the housing (1) for feeding the liquid with volatile substances, wherein the device also comprises a nozzle (14) comprising a dividing element (6) defining a first channel (61) connected to the first air outlet (4) and a second channel (62) connected to the second air outlet (5); and a capillary element (13) that extends from inside the container (10) to the nozzle (14), feeding the liquid with volatile substances from the container (10) to the nozzle (14).

## Description

The present invention relates to a device for diffusing volatile substances, in particular, comprising a fan which allows to create a high pression region inside the device and still obtaining a high-speed airstream egressing the device to diffuse the volatile substances quickly.

### Background of the invention

Existing fragrance delivery devices often result in uneven distribution or localized concentrations of the fragrance, failing to achieve uniform and widespread coverage throughout the desired area, especially when the room is large.

Traditional approaches face challenges in effectively nebulizing the fragrance into fine droplets, and they require multiple moving parts for atomizing and effectively quickly diffuse the active agent in big rooms, for example, combinations of pumps and fan, piezoelectric actuators and fan, heating element and fan, etc.

Other solutions suffer from inefficient atomization or evaporation of the active liquid, leading to undesirable accumulation or pooling of the substance around the outlet area, thereby compromising performance and potentially causing hygiene or safety issues.

### Description of the invention

Therefore, an objective of the present invention is to provide a device for diffusing volatile substances that relies on only one moving element to effectively eject the liquid, atomize it, and effectively diffuse it.

The device for diffusing volatile substances of the invention solves the above-mentioned disadvantages and has other advantages which will be described below.

The device for diffusing volatile substances according to the present invention is described in claim 1, and the dependent claims include additional features that are optional.

In particular, the device for diffusing volatile substances comprises:
- a housing provided with an air inlet and a first and second air outlets;
- a fan placed inside the housing for directing air from the air inlet to the air outlets; and
- a container housing liquid with volatile substances, the container being connected to the housing for feeding the liquid with volatile substances;
- a nozzle comprising a dividing element defining a first channel connected to the first air outlet and a second channel connected to the second air outlet; and
- a capillary element that extends from inside the container to the nozzle, feeding the liquid with volatile substances from the container to the nozzle.

According to a preferred embodiment, the capillary element is a capillary tube.

Furthermore, the first channel has preferably a frustoconical shape, with its wider end being oriented towards the fan and its narrower end being oriented towards the first outlet, and the second channel has substantially the same width along its whole length.

Preferably, the second channel surrounds the first channel, i.e., the first channel is an inner channel, and the second channel is an outer channel.

According to a preferred embodiment, the fan is an axial fan, for example, a brushless axial fan.

Furthermore, the container is preferably provided with a cap that seals the container, the cap comprising an opening for the passage of the capillary element.

According to a preferred embodiment, the capillary element is surrounded by a cover between the container and the housing.

Furthermore, the first and second channels preferably define an annular section, and the first channel has a diameter at its end closer to the first outlet that is at least 5 times smaller than the diameter of the second channel.

Preferably, the dividing element comprises a plurality of ribs between the first and second channels.

Using a fan to pressurize the container, atomize the liquid, and disperse the treated air to obtain wide coverage in a short time.

Furthermore, the dividing element allows to create a high pression region inside of the housing and still obtain a high-speed airstream that exits the housing to diffuse the substance quickly.

Furthermore, the first and second channels reduce the droplet size and ensure high-speed transport of the volatile substances, in a way that the treated air covers a room very quickly.

### Brief description of the drawings

For a better understanding of what has been explained above, some drawings are included in which, schematically and only by way of a non-limiting example, a practical case of embodiment is represented.
Figure 1 is a perspective view of the device for diffusing volatile substances according to the present invention;
Figure 2 is a sectional elevation view of the device for diffusing volatile substances according to the present invention.

### Description of a preferred embodiment

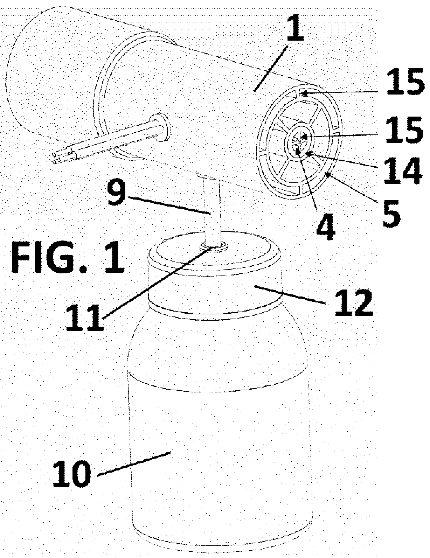

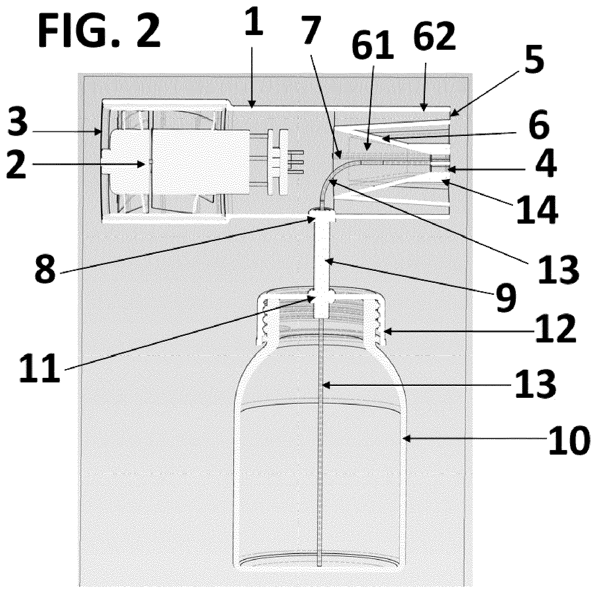

Figures 1 and 2 show the device for diffusing volatile substances according to the present invention.

The device comprises a housing (1), which preferably is substantially cylindrical and encloses a fan (2), which is preferably a brushless axial fan.

The housing (1) can further comprise more openings for electrical connections to power the fan (2), not shown in the drawings.

The housing (1) comprises an air inlet (3) located at a first end of the housing, near the fan (2) and allows the air to enter the housing (1). As shown in Fig. 2, the fan (2) is placed towards the air inlet (3), so that the air that enters the housing (1) is directed to the fan (2).

The housing (1) also houses a nozzle (14) comprising a first and second air outlets (4, 5) which are located at a second end of the housing (1), which allow the air propelled by the fan (2) to exit the housing (1).

This nozzle (14) also comprises a dividing element (6) that splits the airflow and divides in two, guiding it to the first and second air outlets (4, 5) through a first and second channels (61, 62).

As shown in Fig. 2, the first channel (61) has a frustoconical shape, with its wider end oriented towards the fan (2) and its narrower end oriented towards the first outlet (4). This design restricts the air flow enough to reduce the velocity of the airflow and creates a static high-pressure zone (7) in the surrounding area.

According to the embodiment shown, the second air outlet (5) is substantially annular and surrounds the first air outlet (4), and the second channel (62) has substantially constant cross-section, so the air flowing through it unrestricted, with virtually no reduction in its speed.

The housing (1) also comprises an opening (8) for receiving an end of a cover (9) that communicates the high-pressure zone (7) with the interior of a sealed container (10) containing the liquid with the volatile substances.

The sealed container (10) also comprises a cap (12) provided with an opening (11) and a capillary element (13), such as a capillary tube. This capillary element (13) is partially submerged in the liquid with the volatile substances, and it is connected with the nozzle (14), so that it extends up to the first air outlet (4), as shown in Fig. 2.

According to a preferred embodiment, the capillary element (13) has an inner diameter from 0.1 to 1.5 mm, preferably 0.2 mm.

Furthermore, according to the embodiment shown, the capillary element (13) passes through the cover (9) inside the housing (1).

In an alternative embodiment, not shown the capillary element (13) can go through extra openings in the cap (12) and the housing (1).

Furthermore, as shown in Fig. 1, the dividing element (6) comprises a plurality of ribs (15) between the first and second channels (61, 62).

Just as an example, the first and second channels (61, 62) define a circular section, and the first channel (61) has a diameter at its end closer to the first outlet (4) that is at least 5 times smaller than the diameter of the second channel (62). For example, the diameter of the first channel (61) is 5 mm, and the diameter of the second channel (62) is 27 mm.

Furthermore, the fan (2) has a diameter between 20 and 120 mm, preferably 30 mm, and the expelled air travels at a speed greater than 20 m/s.

The operation of the device for diffusing volatile substances is the following.

The fan (2) operates at very high velocity, for example, 50,000 to 150,000 rpm, taking air from the environment and feeding the housing (1), and elevating the air pressure in the high-pressure zone (7).

This pressure is transmitted to the sealed container (10), creating static pressure strong enough to deliver the fragrance through the capillary element (13), forcing it out of the nozzle (14) and atomizing it into the air stream coming out through the first air outlet (4).

The air flow exiting from the second outlet (5) reduces the droplet size, promotes evaporation and further disperses the volatile substances into the environment ensuring wide coverage and preventing liquid depositions in the surroundings of the device.

Although reference has been made to a specific embodiment of the invention, it is obvious to a person skilled in the art that the device for diffusing volatile substances described is susceptible to numerous variations and modifications, and that all the details mentioned can be replaced by technically equivalent ones, without departing from the scope of protection defined by the appended claims.

## Claims

1. Device for diffusing volatile substances, comprising:
- a housing (1) provided with an air inlet (3) and a first and second air outlets (4, 5);
- a fan (2) placed inside the housing (1) for directing air from the air inlet (3) to the air outlets (4, 5); and
- a container (10) housing liquid with volatile substances, the container (10) being connected to the housing (1) for feeding the liquid with volatile substances, **characterized in that** the device also comprises:
- a nozzle (14) comprising a dividing element (6) defining a first channel (61) connected to the first air outlet (4) and a second channel (62) connected to the second air outlet (5); and
- a capillary element (13) that extends from inside the container (10) to the nozzle (14), feeding the liquid with volatile substances from the container (10) to the nozzle (14).

2. Device for diffusing volatile substances according to claim 1, wherein the capillary element (13) is a capillary tube.

3. Device for diffusing volatile substances according to claim 1, wherein the first channel (61) has a frustoconical shape, with its wider end being oriented towards the fan (2) and its narrower end being oriented towards the first outlet (4).

4. Device for diffusing volatile substances according to claim 1, wherein the second channel (62) has substantially the same width along its whole length.

5. Device for diffusing volatile substances according to claim 1, wherein the second channel (62) surrounds the first channel (61).

6. Device for diffusing volatile substances according to claim 1, wherein the fan (2) is an axial fan.

7. Device for diffusing volatile substances according to claim 1, wherein the container (10) is provided with a cap (12) that seals the container (10), the cap (12) comprising an opening (11) for the passage of the capillary element (13).

8. Device for diffusing volatile substances according to claim 1, 2 or 7, wherein the capillary element (13) is surrounded by a cover (9) between the container (10) and the housing (1).

9. Device for diffusing volatile substances according to claim 1 or 3, wherein the first and second channels (61, 62) define a circular section, and the first channel (61) has a diameter at its end closer to the first outlet (4) that is at least 5 times smaller than the diameter of the second channel (62).

10. Device for diffusing volatile substances according to claim 1, wherein the dividing element (6) comprises a plurality of ribs (15) between the first and second channels (61, 62).
